(19) Europäisches Patentamt
European Patent Office
Office européen des brevets

(11) **EP 4 663 234 A1**

(12) **EUROPEAN PATENT APPLICATION**

(43) Date of publication:
**17.12.2025 Bulletin 2025/51**

(21) Application number: **24315291.5**

(22) Date of filing: **14.06.2024**

(51) International Patent Classification (IPC):
**A61N 1/37** (2006.01)     **A61N 1/08** (2006.01)
**A61N 1/36** (2006.01)     **G01R 33/28** (2006.01)
**A61B 5/055** (2006.01)

(52) Cooperative Patent Classification (CPC):
**A61N 1/3718; A61B 5/055; A61N 1/08;**
**A61N 1/36142; G01R 33/288**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Sorin CRM SAS**
**92140 Clamart (FR)**

(72) Inventor: **SETZU, Romano**
**78000 Versailles (FR)**

(74) Representative: **Page, White & Farrer Germany LLP**
**Widenmayerstraße 10**
**80538 München (DE)**

(54) **SATURATION-BASED DISCRIMINATION OF MAGNETIC FIELD STRENGTHS IN ACTIVE IMPLANTABLE MEDICAL DEVICES**

(57) This invention relates to an active implantable medical device that is configured to discriminate weak magnetic fields (e.g., for entering a magnet mode) from strong magnetic fields (e.g., for entering an MRI mode) without introducing additional components, by exploiting a saturation effect of an inductance that depends on the magnetic field strength and using the saturation-dependent inductance as frequency-determining element of a resonance-based signal generator such as an oscillator. The discrimination can then be achieved by comparing the resonance-based frequency with a predetermined reference frequency.

**Fig. 4**

## Description

FIELD OF THE INVENTION

The invention relates to techniques for protecting active implantable medical devices (e.g., as defined by Directive 90/385/EEC of 20 Jun. 1990 of Council of European Communities), such as generators and their associated sensors, when a patient is subject to an examination by magnetic resonance imaging (MRI) equipment.

BACKGROUND OF THE INVENTION

Active implantable medical devices comprise a housing containing various electronic circuits and a battery, generally referred to as a generator, that is electrically and mechanically connected to one or more leads with electrodes. The leads are intended to get into contact with a tissue of the patient, e.g., the myocardium, at sites where electrical potentials can be collected (i.e., detected) and/or stimulation pulses can be applied (i.e., delivered).

MRI examination has been contraindicated for patients with an implanted cardiac pacemaker or defibrillator type of generator. Several types of problems arise under this situation, such as RF-induced heating near the electrodes connecting the generator to the patient's heart, forces and torques of attraction exerted on the device immersed in the high intensity static magnetic field produced by the MRI equipment during an examination, and/or unpredictable behavior of the device itself due to the exposure to the magnetic fields of the MRI equipment.

In the absence of special precautions, the problems that are likely to affect the behavior of the implantable medical device under an MRI examination include an erratic detection of a strong static field generated by MRI equipment, especially in devices equipped with a low-magnetic-field sensor (e.g., a Reed switch) which are meant to detect handheld magnets but which are not able to distinguish from high MRI static fields. The handheld magnets (permanent magnets) can be used by a practitioner to put the device in a safe operating or "magnet mode", for example, when using an electric scalpel or when evaluating battery depletion of the device. In the magnet mode, the stimulation frequency may be fixed and may reflect the level of battery charge.

The low-magnetic-field sensors are designed to detect static magnetic fields of relatively low intensity but are likely to exhibit a totally unpredictable behavior in an MRI examination environment where the magnetic fields are often thousands of times stronger than that of a permanent magnet. Problems also may include deterioration of the intrinsic performance of the device, and misinterpretation of the dynamic signals emitted by the MRI equipment by the device as cardiac signals, including, for example, an inhibition of the stimulation function by the dynamic signals emitted by the MRI equipment that are inadvertently detected by the device as cardiac signals.

Throughout the duration of an MRI examination-which can last several minutes-the device should nevertheless remain functional and provide if necessary seamless and predictable stimulation to the patient's myocardium.

It is therefore desirable to have means for detecting and means for managing such a situation, e.g., providing functions such as indicating to the device that the patient will be subjected to an MRI examination, inhibiting the circuits of the device that may be disturbed by the electromagnetic fields emitted by the MRI equipment, and operating the device in a dedicated pacing mode, tailored to the patient and compatible with the electromagnetic fields emitted by the MRI equipment.

In conventional implantable medical devices, there is no way to detect strong static magnetic fields and hence automatically detect entrance into an MRI system. Such devices must be programmed manually to an operating mode (hereafter called MRI Mode) which ensures safety of the device during the MRI examination by managing (including deactivating, if needed) all components which may adversely interact with the MRI magnetic fields, including magnet detection sensors. Alternatively, an additional sensor (e.g., a Hall effect magnetic sensor) may be provided to detect the stronger magnetic field generated during MRI examination. In both cases, they need a redesigned hardware that incurs additional cost and creates extra constraints on the circuits against the design requirements for miniaturizing these devices.

SUMMARY OF THE INVENTION

It is an objective of the present invention to discriminate weak (any source) from strong (MRI-based) magnetic fields without requiring hardware modification.

This object is achieved by an implantable medical device as claimed in claim 1, a method as claimed in claim 11, and a computer program product as claimed in claim 12.

According to first aspect, an implantable medical device is provided, which comprises:

a magnetic-field-dependent signal generator configured to generate an oscillation signal with a magnetic-field-dependent frequency, wherein the magnetic-field-dependent signal generator comprises a frequency determining element with a saturation-dependent inductance that depends on a magnetic field strength imposed on the implantable medical device; and
a mode control circuit for comparing a period of the magnetic-field-dependent frequency with different multiples of periods of a reference frequency to discriminate between a weak magnetic field and a strong magnetic field and for setting the implantable medical device to a first operating mode during a discriminated weak magnetic field and to a second operating mode during a discriminated strong magnetic field.

According to a second aspect, a method of setting operating modes of an implantable medical device in dependence on a weak magnetic field and a strong magnetic field is provided, wherein the method comprises:

generating an oscillation signal with a magnetic-field-dependent frequency by using a frequency determining element with a saturation-dependent inductance that depends on a magnetic field strength imposed on the implantable medical device;
comparing a period of the magnetic-field-dependent frequency with different multiples of periods of a reference frequency to discriminate between the weak magnetic field and the strong magnetic field; and
setting the implantable medical device to a first operating mode during a discriminated weak magnetic field and to a second operating mode during a discriminated strong magnetic field.

According to a third aspect, a computer program product is provided, which comprises code means for producing the steps of the above method of the second aspect when run on a microcontroller of an implantable medical device.

Accordingly, weak magnetic fields (e.g., for entering a magnet mode) can be discriminated from strong magnetic fields (e.g., for entering an MRI mode) without introducing additional components, by exploiting a saturation effect of an inductance that depends on the magnetic field strength. The saturation-dependent inductance can be used as frequency-determining element of a resonance-based signal generator such as an oscillator. The discrimination can then be achieved by comparing the resonance-based frequency with a predetermined reference frequency.

According to a first option of any one of the first to third aspects, the saturation-dependent inductance may be comprised in a telemetry antenna of the implantable medical device.

According to a second option which may be combined with the first option, the saturation-dependent inductance may comprise a coil with ferromagnetic core of the telemetry antenna.

According to a third option which may be combined with the first or second option, trimming capacitors of the telemetry antenna may be provided, wherein the trimming capacitors may be dynamically adjustable during operation of the implantable medical device to improve detection sensitivity of the weak and strong magnetic fields.

According to a fourth option which may be combined with any one of the first to third options or any one of the first to third aspects, a reference signal generator (e.g., a quartz oscillator) may be provided, which may be configured to generate a reference signal with the predetermined reference frequency.

According to a fifth option which may be combined with any one of the first to fourth options or any one of the first to third aspects, the mode control circuit may be configured to determine a detection of the weak magnetic field if the length of the period of the magnetic-field-dependent frequency is shorter than the length of a first multiple of periods of the reference frequency, and to determine a detection of the strong magnetic field if the length of the period of the magnetic-field-dependent frequency is shorter than the length of a second multiple of periods of the reference frequency, wherein the first multiple is larger than the second multiple.

According to a sixth option which may be combined with any one of the first to fifth options or any one of the first to third aspects, the strong magnetic field may correspond to a magnetic field imposed during an MRI examination.

According to a seventh option which may be combined with any one of the first to sixth options or any one of the first to third aspects, the weak magnetic field may correspond to a magnetic field imposed on the implantable medical device by approaching the implantable medical device with a permanent magnet to trigger a magnet mode as the first operating mode..

According to an eighth option which may be combined with any one of the first to seventh options or any one of the first to third aspects, the mode switching circuit may be configured to detect the weak magnetic field when the value of the saturation-dependent inductance has decreased by at least 20% due to saturation effects by the impact of the imposed magnetic field strength.

According to a ninth option which may be combined with any one of the first to eighth options or any one of the first to third aspects, a B-field strength of the weak magnetic field may be larger than 1mT and a B-field strength of the strong magnetic field may be larger than 100mT.

It is noted that the above device may be implemented based on discrete hardware circuitries with discrete hardware components, integrated chips, or arrangements of chip modules, or based on signal processing devices or chips controlled by software routines or programs stored in memories, written on a computer readable media, or downloaded from a network, such as the Internet.

It shall be understood that the implantable medical device of claim 1, the method of claim 11, and the computer program product of claim 12 may have similar and/or identical preferred embodiments, in particular, as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically an implantable medical device, in which proposed embodiments can be implemented;
Fig. 2 shows schematically waveforms of a reference oscillator signal and a saturation-dependent resonance signal of a telemetry antenna;
Fig. 3 shows schematically a saturation curve of an inductance of the telemetry antenna and related impact on resonance frequency, saturation and the saturation-dependent resonance signal;
Fig. 4 shows schematically a block diagram of an antenna-based magnetic field strength discrimination circuitry according to a first embodiment; and
Fig. 5 shows a flow diagram of a procedure for magnetic mode switching based on a discrimination of magnetic field strengths, according to a second embodiment.

DETAILED DESCRIPTION OF EMBODIMENTS

Various embodiments of the present invention are now described based on an implantable medical device which provides electrical stimulation therapies, such as a neurostimuator, cardiac pacemaker or implantable cardioverter defibrillator (ICD). Such devices may comprise a programmable microcontroller and/or microprocessor circuitry to receive, format, and process electrical signals collected by implanted electrodes and deliver a therapy via these electrodes. It is possible to receive software by telemetry via a telemetry antenna and to store the software in a memory and execute the software to implement at least some of the functions described in the following embodiments.

Special techniques have been proposed to detect static magnetic fields of an MRI type, with strength in the order of Tesla (typically between 0.5 and 3T; herein referred to as a "strong magnetic field"). In implantable medical devices that are sensitive to the presence of a permanent magnet, a magnetic field detector may have been configured to detect weak magnetic fields (e.g., typically in a range between 1 and 10mT; herein referred to as a "weak magnetic field"). However, such a magnetic field detector is unable to discriminate between the weak magnetic fields and the strong magnetic fields.

It is therefore desirable to configure implantable medical devices to properly discriminate between weak and strong magnetic fields without substantial hardware modification of the implantable medical device.

It is noted that - throughout the present disclosure - only those structural elements and functions are shown and/or described, which are useful to understand the embodiments. Other structural elements and functions are omitted for brevity reasons. Furthermore, the structure and/or function of blocks with identical reference numbers that have been described before are not described again, unless an additional specific functionality is involved.

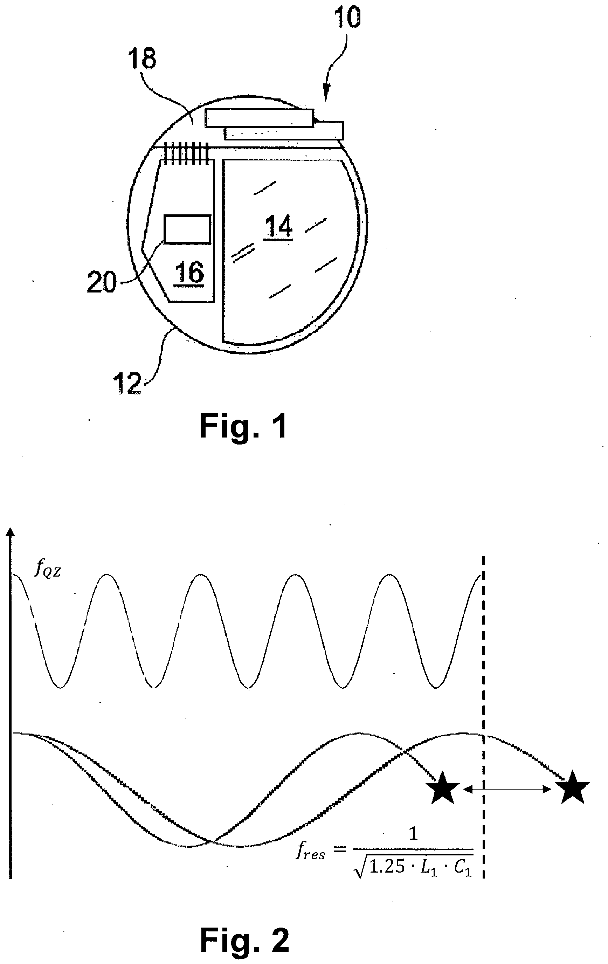

Fig. 1 shows schematically an implantable medical device (IMD), in which proposed embodiments can be implemented.

With reference to Fig. 1, the housing 12 of the IMD 10 contains a battery 14 and an electronic circuitry 16. The electronic circuitry 16 may be connected to a connector block 18 that removably receives one or more leads (not shown) having electrodes for detection and/or stimulation.

The IMD 10 further comprises a telemetry circuitry (e.g., as part of the electronic circuitry 16) and a telemetry antenna 20. Programming commands or data can be transmitted during uplink or downlink telemetry between the IMD telemetry circuitry and an external telemetry circuitry included in a programmer or monitoring unit (not shown). For example, the IMD telemetry circuitry may require the use of an external programming head containing an external antenna to be positioned over the IMD 10.

The telemetry antenna 20 of the IMD 10 may thus be used for transmitting programming commands or data between the IMD telemetry antenna 20 and an external telemetry antenna (not shown) associated with the external programmer using inductive telemetry transmission (which is based on a low-frequency, short-distance inductive communication) .In an uplink telemetry transmission, the external telemetry antenna operates as a telemetry receiver antenna, and the IMD telemetry antenna 20 operates as a telemetry transmitter antenna. Conversely, in a downlink telemetry transmission, the external telemetry antenna operates as a telemetry transmitter antenna, and the IMD telemetry antenna 20 operates as a telemetry receiver antenna. Both telemetry antennas are coupled to transceiver circuitry including a transmitter and a receiver.

The IMD telemetry antenna 20 is generally designed for efficient, reliable telemetry transmission in the implanted environment. It may be located within the housing 12 (which may be a hermetic housing containing the device circuitry).

In order to reduce space requirements of a coil of the telemetry antenna 20 and further increase its efficiency, the coil may be constructed with a core made of ferrite or other magnetically permeable or ferromagnetic material.

The coil with a ferrite core produces increased magnetic flux that can be linked with the external telemetry antenna of the external programmer, which allows the use of a smaller coil within the header to achieve satisfactory performance. A ferrite-core coil, for example, needs to be only approximately one-third the diameter of an air-core coil to achieve similar performance.

The electronic circuitry 16 of the IMD 10 may generally include timing and control circuitry and an operating system that may employ a microprocessor or a digital state machine for timing sensing and therapy delivery functions in accordance with a programmed operating mode. The microprocessor and an associated memory may be coupled to various components of the IMD 10, e.g., via a data/address bus. Furthermore, a therapy delivery unit may be provided for delivering a therapy, such as an electrical stimulation, under the control of the timing and control circuitry. In the case of electrical stimulation therapies, such as cardiac stimulation therapies, the therapy delivery unit may be coupled to two or more electrodes via a switch matrix which can be used for selecting electrodes and corresponding polarities for delivering electrical stimulation pulses.

The electrodes may also be used for sensing electrical signals within the body, such as cardiac signals or other electromyogram signals, or for measuring impedance. In the case of cardiac stimulation devices, cardiac electrical signals may be sensed for determining when an electrical stimulation therapy is needed and in controlling the timing of stimulation pulses. The electrodes may generally be carried on one or more leads coupled to IMD the 10 as described above.

The IMD 10 may additionally or alternatively be coupled to one or more physiological sensors. Such sensors may include pressure sensors, accelerometers, flow sensors, blood chemistry sensors, activity sensors or other physiological sensors. These sensors may be coupled to the IMD 10 via a sensor interface which provides sensor signals to the electric circuitry 16. Sensor signals may be used by the microprocessor for detecting physiological events or conditions. For example, the IMD 10 may monitor heart wall motion, blood pressure, blood chemistry, respiration, or patient activity. Monitored signals may be used for sensing the need for delivering a therapy under control of the operating system.

The operating system may comprise associated memory for storing a variety of programmed-in operating mode and parameter values that are used by the microprocessor. Moreover, the associated memory may be used for storing data compiled from sensed physiological signals and/or relating to device operating history for telemetry out on receipt of a retrieval or interrogation instruction.

In embodiments, the IMD 10 is configured to discriminate weak static magnetic fields from strong magnetic fields without introducing additional components but relying on the existing telemetry antenna 20 as frequency-determining element of signal generator such as an oscillator.

In the presence of a detected strong magnetic field (e.g., at the beginning of an MRI examination), the microcontroller of the IMD 10 switches to an MRI-safe mode (referred to as MRI mode) that is compatible with a strong magnetic field for the duration of the MRI examination. Once the MRI examination is completed, the IMD 10 may be reset to a standard mode of operation (referred to as standard mode).

Furthermore, upon detecting a weak magnetic field, the microcontroller sets the IMD 10 into a magnet mode, as specified by the manufacturer of the IMD 10. In an example, a patient wearing the IMD 10 may be paced in a conventional DOO mode with a stimulation frequency that is determined based on the level of battery depletion (which allows testing of that level). The DOO mode means dual pacing ("D") in atrium (A) and ventricle (V) with switched off ("O") sensing and response to sensing. This mode results in AV sequential pacing at a lower rate limit regardless of the heart's own intrinsic activity. The DOO mode is an asynchronous pacing and is usually used only in certain situations, such as when a magnet is placed over the IMD 10 or sometimes when a patient is having surgery. Other conventional asynchronous pacing modes can be used as deemed appropriate for the device used and the patient, e.g., VOO, AOO.

It is to be noted that the above asynchronous mode proportional to the battery level applies to bradycardia devices only. In other CRM devices, the magnet mode can be implemented very differently in dependence on whether the CRM device treats bradycardia (e.g., pacemakers, CRT-Ps) or tachycardia/dyssynchronization (e.g., ICDs, CRT-Ds). Tachycardia devices would not implement a battery-related asynchronous rhythm but would rather deactivate all HV therapies.

When the magnetic field is removed, the IMD 10 may return to the programmed standard mode of operation, which may comprise a number of different standard operating modes responsive to the patient's condition. In case of weak magnetic fields, the return switch can happen fast (e.g., a few seconds) because reversion to normal therapies is favored. In case of strong magnetic fields (e.g., as during an MRI examination), the return switch can happen after a longer waiting time (e.g., a few minutes) to make sure that the patient has left the MRI room (safety is favored against MRI hazards). The telemetry antenna 20 with the ferromagnetic core can be interpreted in electrical terms as an LC resonance circuit (as frequency-determining element) with a resonance frequency that can be calculated as

$$fres = 1/(2\pi\sqrt{LC})$$

The following exemplary equation is relevant to a specific circuit implementation with two capacitances C1 and C2 described below:

$$f_{res} = \frac{1}{\sqrt{1.25 \cdot L_1 \cdot C_1}}$$

wherein L1 denotes the nominal value of an inductive component of the coil antenna in an unsaturated state. In an example, the nominal value may be about 100 mH. Furthermore, C1 designates a serial capacitor used for tuning the telemetry antenna 20. C1 may be calibrated during production to properly discriminate between detection of weak and strong magnet fields. Furthermore, in the above equation, an additional parallel capacitor C2 is calibrated relatively to the serial capacitor C1.

The presence of a magnetic field with sufficient strength has the effect of saturating the core of the inductive component L1 (ferromagnetic core) of the telemetry antenna 20. In an example, C1 may be calibrated such that a magnetic field is detected for a minimum of a 20% decrease of the value of the inductance L1 due to saturation effects by the impact of the external magnetic field.

Fig. 2 shows schematically a time diagram with an upper waveform of a reference oscillator signal (e.g., with reference frequency $f_{ref}$=30000 Hz) with a length of five oscillation periods and two lower waveforms of a saturation-dependent resonance signal (e.g., with the above resonance frequency $f_{res}$ generated by a telemetry antenna (e.g., the telemetry antenna 20 of Fig. 1) as frequency-determining element of a frequency generator (e.g., oscillator) with and without saturation by a prevailing magnetic field.

The star-shaped marker indicates the end of one oscillation period of the saturation-dependent resonance signal. As can be gathered from Fig. 2, the resonance signal without magnetic field has a longer oscillation period (lower resonance frequency) that ends after the threshold (dashed line) of five reference oscillation periods, while the resonance signal with saturating magnetic field has a shorter oscillation period (higher resonance frequency) that ends before the threshold (dashed line) of five reference oscillation periods. Thus, if the zero crossing after one period of the resonance oscillation signal occurs after five periods of the reference oscillation signal (e.g., quartz periods), no magnetic field is detected. Otherwise, if the zero crossing after one period of the resonance oscillation signal occurs before five reference oscillation periods, presence of a magnetic field is detected.

As a result, the change of the inductive component L1 due to saturation effects leads to a change of the resonance frequency $f_{res}$ of the telemetry antenna 20. Thus, (static) magnetic fields can be detected in the IMD 10 by comparing the period of a frequency generator with telemetry antenna as frequency-determining element (e.g., an LC oscillator including the saturating inductor of the telemetry antenna 20) with a predetermined number of periods (e.g., five periods) of a reference oscillator (e.g., quartz oscillator of the with fixed reference frequency $f_{QZ}$= $f_{ref}$= 30000 Hz) of the timing and control circuitry provided in the IMD 10.

In embodiments, the predetermined number of periods of the reference oscillator may be modified and determined to discriminate weak magnetic field (leading to a magnetic flux density or B-field strength above e.g. 1mT in the core of the telemetry antenna 20) from strong (e.g., MRI-based) magnetic fields (leading to a magnetic flux density or B-field strength above e.g. 100mT in the telemetry antenna 20).

It is therefore proposed to modulate the number of reference periods (e.g., quartz periods) to be used for the comparison of the length of the reference oscillation periods with the saturation-based period(s) of the antenna-based resonance frequency.

As example, for shorter reference oscillation periods (e.g., four periods and below) the microcontroller of the IMD 10 can detect higher inductor saturation levels (e.g., caused during MRI examination), which are directly proportional to the magnetic field intensity.

Thus, in embodiments, by calibrating the telemetry antenna saturation levels based on a target intensity of the magnetic field, two or more different saturation levels (low and high, low and medium and high, etc.) corresponding to different target magnetic field levels.

The trimming capacitors C1 and C2 of the telemetry antenna 20 can also be adjusted dynamically during operation of the IMD 10 to improve detection sensitivity through inductance saturation.

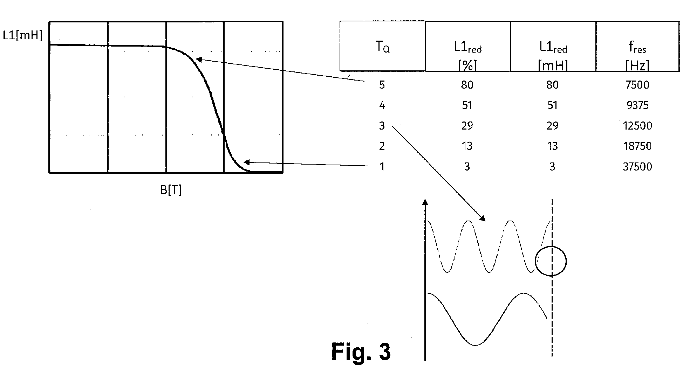

Fig. 3 shows schematically an exemplary saturation curve (left portion of Fig. 3) of an inductance L1 [mH] of the telemetry antenna (e.g., the telemetry antenna 20 of Fig. 1) in dependance of the magnetic field strength B[T] and related impact on resonance frequency, saturation and the saturation-dependent resonance signal (right portion of Fig. 3). The values in the parameter table are given for constant C1/C2 values.

As can be gathered from the saturation curve and the parameter table of Fig. 3 (for an exemplary case of a reference frequency (quartz frequency) of 30kHz and an exemplary inductance L1 of 100mH), a 20% saturation-dependent reduction (weak magnetic field) of the inductance L1 to a reduced inductance $L1_{red}$=80% which corresponds to e.g. $L1_{sat}$=80mH leads to a resonance frequency of e.g. 7500Hz that can be measured with a measurement test period $T_Q$ of five periods (upper arrow). On the other hand, a 97% saturation-dependent reduction (strong magnetic field) of the inductance L1 to a reduced inductance $L1_{red}$=3% which corresponds to e.g. $L1_{sat}$=3mH leads to a resonance frequency of e.g. 37500Hz that could be measured with a measurement test period $T_Q$ of only one period (lower arrow).

A medium example for strong magnetic field detection is shown with the lower right-hand waveform diagram of Fig. 3, where three test periods are used for detecting a 71% saturation-dependent reduction of the inductance L1 to a reduced inductance $L1_{red}$=29% which corresponds to e.g. $L1_{sat}$=29mH at a resonance frequency of 12500Hz.

Further examples for two and four test periods are shown in the parameter table of Fig. 3.

In the exemplary saturation curve of Fig. 3, the inductance L1 fully saturates at a magnetic D-field level of around 0.75 T.

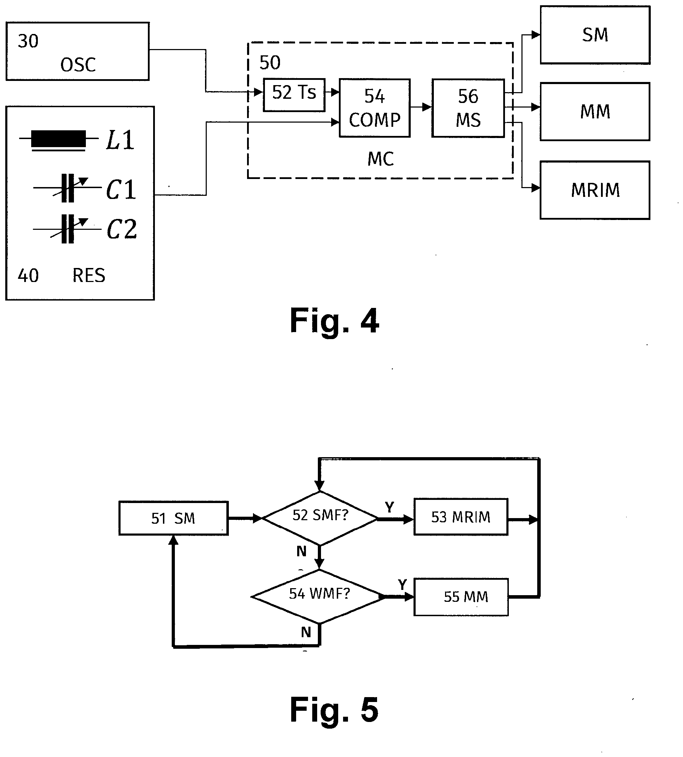

In embodiments, the proposed automatic discrimination between weak and strong magnetic fields can be implemented in specific IMDs or implanted pulse generators (IPGs) by characterizing (e.g., measuring, plotting etc.) the inductance curve (e.g., telemetry antenna saturation curve) against static magnetic field intensity) to find a suitable spot for consistently detecting a strong magnetic field of 100mT (e.g., for MRI detection) and then developing a processor-based control function to implement a dynamic change of reference test periods (e.g., quartz periods) used for magnet field detection. This may be achieved by a procedure (algorithm) for combining magnet detection (low magnetic field) and MRI detection (strong magnetic fields), as described below in connection with the embodiments of Figs. 4 and 5.

Fig. 4 shows schematically a block diagram of an antenna-based magnetic field strength discrimination circuitry according to a first embodiment, which may be implemented in the electronic circuit 16 of the IMD 10 of Fig. 1.

A reference oscillator (OSC) 30 which may be a quartz oscillator or other type of fixed-frequency oscillator, and which may be provided in the timing and control circuitry of the IMD 10 of Fig. 1, is connected to a mode control circuit (MC) 50 (which may be implemented by a software-controlled microcontroller or microprocessor or an (integrated) hardware circuit (e.g., application-specific integrated circuit (ASIC) or programmable gate array (PGA) circuit) of the IMD 10, to supply a reference frequency.

Additionally, a resonance oscillator circuit (RES) 40 is connected to the mode control circuit 50, which uses the saturation-dependent inductance L1 of the telemetry antenna 20 of the IMD 1 together with at least one of the trimming capacitances C1 and C2 and optional other capacitors as frequency-determining LC element to generate a resonance frequency which depends on the saturation level of the inductance L1 of the telemetry antenna.

The mode control circuit 50 may comprise an oscillation period selection function (Ts) 52 for selecting a proper number of oscillation periods of the reference oscillator 30 based on the magnetic field strength to be discriminated, a comparison circuit or function (COMP) 54 that is configured to compare the length of the selected number of oscillation periods of the reference oscillator 30 with the length of one or more oscillation periods of the resonance oscillator circuit 40. In an example, the oscillation period selection function (Ts) 52 may be implemented by a programmable or fixed look-up table that associates respective oscillation period numbers to specific (e.g., weak and strong) magnetic field strengths. Furthermore, the comparison circuit or function. 54 may be implemented by a timer function which is set and/or reset by the reference oscillator signal and that counts the length of the selected predetermined number of oscillation cycles. Furthermore, the timer function may be configured to be set and/or reset by the resonance oscillator signal and to count the length of the one or more oscillation cycles of the resonance oscillator signal.

The comparison circuit or function 54 may be configured to compare the above counting results and to supply the comparison result to a mode switching circuit or function (MS) 56 which sets or resets different operating modes (including the standard mode (SM), the magnet mode (MM) and the MRI mode (MRIM) described above) of the IMD 10 based on the comparison result.

In the first embodiment, the mode control circuit 50 may be configured to use two different detection criteria for discriminating a low magnetic level for magnet detection and setting the IMD 10 into the magnetic mode and for discriminating a high magnetic level for MRI detection and setting the IMD 10 into the MRI mode. To set the MRI detection, the number of reference oscillation periods may be set to less than five periods, so that an L1 saturation value greater than 20% can be detected. This corresponds to detecting a strong magnetic B-field (e.g., higher than 100mT).

In a modification of the first embodiment, the discrimination between weak and strong magnetic fields (e.g., magnet and/or MRI detection test) may be adjusted by trimming one or both of the capacitors C1 and C2 of the telemetry antenna 20.

Fig. 5 shows a flow diagram of a procedure for magnetic mode switching based on a discrimination of magnetic field strengths, according to a second embodiment. The procedure may be implemented as a software routine that controls a mode switching function of the microcontroller of the IMD 10 of Fig. 1 or the mode control circuit 50 of Fig. 4.

In the second embodiment, a two-states detection procedure combining weak magnetic field detection (for setting the magnet mode) and strong magnetic field detection (for setting the MRI mode) allows a fully automatic switch between these two modes according to the detected field intensity without the need of additional hardware components.

In step 51, the IMD 10 is set into the standard (nominal) mode (SM), e.g., as an initial state after a power-on switch. Then, in step 52 (SMF?), the length (duration) of a selected predetermined shorter reference period (e.g., four or less oscillation periods of a reference oscillation) is compared with the length (duration) of a single oscillation period of a resonance oscillation determined by the saturation-dependent inductance (e.g., the inductance L1 of the telemetry antenna 20), where it is checked if a strong magnetic field (e.g., >100mT) has been detected. I.e., if the shorter reference period is longer or not. If the shorter reference period is longer ("Y"), the procedure continues with step 53 and controls the IMD 10 to enter into the MRI mode (MRIM). If not ("N"), the procedure continues with step 54 and checks for a weak magnetic field.

In step 54 (WMF?), the procedure compares the length (duration) of a newly selected predetermined longer reference period (e.g., five oscillation periods of the reference oscillation) with the length (duration) of a single oscillation period of the resonance oscillation determined by a saturation-dependent inductance (e.g., the inductance L1 of the telemetry antenna 20) and checks if a weak magnetic field (e.g., >1mT) has been detected. I.e., if the longer reference period is longer or not. If the longer reference period is longer ("Y"), the procedure continues with step 55 and controls the IMD 10 to enter into the magnet mode (MM). If not ("N"), the procedure jumps back to step 51 and the IMD 10 remains in the standard mode.

In steps 53 and 54, the procedure may be configured to automatically quit the magnet mode after a predetermined first delay of e.g. 4s or, respectively, automatically quit the MRI mode after a predetermined second delay of 5min, and then jump-back to step 52 and repeat the checking process of steps 52 and 54. Optionally, no additional check may be performed and the procedure may jump back to step 51 and enter the initial standard mode.

According to a modified embodiment, the procedure may be configured to continuously monitor for a "weak field" presence (step 54). If no weak magnetic field is detected for a predetermined time period (e.g., at least 4s), the procedure jumps back to standard mode (step 51).

According to another modified embodiment, the presence of any magnetic field could be checked during the MR examination once the MRI mode (step 53) has been entered. In this case, the strong field test (step 52) is only done to trigger an entrance to the MRI Mode (step 53).

According to another modified embodiment, the procedure may start with step 54 (check for weak magnetic field) after the initial step 51 (standard mode) and may then enter the magnet mode (step 55), if affirmative. If not, the procedure may jump back to the initial step 51. In the magnet mode, the procedure may continue with step 52 (check for strong magnetic field) and may then enter the MRI mode (step 53), if affirmative. If not, the procedure may jump back to step 54 (check for weak magnetic field). In the MRI mode, the procedure may check again for the strong magnetic field and may stay in the MRI mode, if affirmative. If not, the procedure may jump back to the initial step 51 and enter the standard mode.

In other embodiments, the saturation-dependent inductance L1 may be provided as a separate element that is not part of a telemetry antenna. E.g., in IMDs or IPGs without telemetry antenna or with other types of telemetry antennas that do not provide a saturation-dependent inductance.

To summarize, an active implantable medical device has been described, that is configured to discriminate weak magnetic fields (e.g., for entering a magnet mode) from strong magnetic fields (e.g., for entering an MRI mode) without introducing additional components, by exploiting a saturation effect of an inductance that depends on the magnetic field strength and using the saturation-dependent inductance as frequency-determining element of a resonance-based signal generator (resonance oscillator). The discrimination can then be achieved by comparing the resonance-based frequency with a predetermined reference frequency.

While the invention has been illustrated and described in detail in the drawings and foregoing description, such illustration and description are to be considered illustrative or exemplary and not restrictive. The invention is not limited to the disclosed embodiments. The proposed discrimination between weak and strong magnetic fields can be achieved by any kind of frequency comparison in the analog or digital (after analog-to-digital (A/D) conversion) time domain or in the analog or digital frequency domain. In the time domain, A/D converted oscillator signals may be compared by digital signal processing to decide about strong or weak magnetic field strengths. In the frequency domain, the reference oscillator signal may be mixed with the resonance oscillator signal to obtain a differential output frequency that directly reflects the strength of the prevailing magnetic field.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. The foregoing description details certain embodiments of the invention. It will be appreciated, however, that no matter how detailed the foregoing appears in the text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

The described operations or procedures like those indicated in Fig. 5 can be implemented as program code means of a computer program and/or as dedicated hardware of the receiver devices or transceiver devices, respectively. The computer program may be stored and/or distributed on a suitable medium, such as an optical storage medium or a solid-state medium, supplied together with or as part of other hardware, but may also be distributed in other forms, such as via the Internet or other wired or wireless telecommunication systems.

**Claims**

1. An implantable medical device (10) comprising:

    a magnetic-field-dependent signal generator (40) configured to generate an oscillation signal with a magnetic-field-dependent frequency, wherein the magnetic-field-dependent signal generator (40) comprises a frequency determining element with a saturation-dependent inductance (L1) that depends on a magnetic field strength imposed on the implantable medical device (10); and
    a mode control circuit (50) for comparing a period of the magnetic-field-dependent frequency with different multiples of periods of a reference frequency to discriminate between a weak magnetic field and a strong magnetic field and for setting the implantable medical device (10) to a first operating mode during a discriminated weak magnetic field and to a second operating mode during a discriminated strong magnetic field.

2. The device of claim 1, wherein the saturation-dependent inductance (L1) is comprised in a telemetry antenna (20) of the implantable medical device (10).

3. The device of claim 2, wherein the saturation-dependent inductance (L1) comprises a coil with ferromagnetic core of the telemetry antenna (20).

4. The device of claim 2 or 3, further comprising trimming capacitors (C1, C2) of the telemetry antenna (20), wherein the trimming capacitors (C1, C2) are dynamically adjustable during operation of the implantable medical device (10) to improve detection sensitivity of the weak and strong magnetic fields.

5. The device of any one of the preceding claims, further comprising a reference signal generator (30), in particular a quartz oscillator, configured to generate a reference signal with the predetermined reference frequency.

6. The device of any of the preceding claims, wherein the mode control circuit (50) is configured to determine a detection of the weak magnetic field if the length of the period of the magnetic-field-dependent frequency is shorter than the length of a first multiple of periods of the reference frequency, and to determine a detection of the strong magnetic field if the length of the period of the magnetic-field-dependent frequency is shorter than the length of a second multiple of periods of the reference frequency, wherein the first multiple is larger than the second multiple.

7. The device of any one of the preceding claims, wherein the strong magnetic field corresponds to a magnetic field imposed during a magneto resonance imaging, MRI, examination.

8. The device of any one of the preceding claims, wherein the weak magnetic field corresponds to a magnetic field imposed on the implantable medical device (10) by approaching the implantable medical device (10) with a permanent magnet to trigger a magnet mode as the first operating mode.

9. The device of any one of the preceding claims, wherein the mode switching circuit (50) is configured to detect the weak magnetic field when the value of the saturation-dependent inductance (L1) has decreased by at least 20% due to saturation effects by the impact of the imposed magnetic field strength.

10. The device of any one of the preceding claims, wherein a B-field strength of the weak magnetic field is larger than 1mT and a B-field strength of the strong magnetic field is larger than 100mT.

11. A method of setting operating modes of an implantable medical device (10) in dependence on a weak magnetic field and a strong magnetic field, the method comprising:

    generating an oscillation signal with a magnetic-field-dependent frequency by using a frequency determining element with a saturation-dependent inductance (L1) that depends on a magnetic field strength imposed on the implantable medical device (10);
    comparing a period of the magnetic-field-dependent frequency with different multiples of periods of a reference frequency to discriminate between the weak magnetic field and the strong magnetic field; and
    setting the implantable medical device (10) to a first operating mode during a discriminated weak magnetic field and to a second operating mode during a discriminated strong magnetic field.

12. A computer program product comprising code means for generating the steps of claim 11 when run on a microcontroller (50) of an implantable medical device (10).

**Fig. 1**

$$f_{res} = \frac{1}{\sqrt{1.25 \cdot L_1 \cdot C_1}}$$

**Fig. 2**

| $T_Q$ | $L1_{red}$ [%] | $L1_{red}$ [mH] | $f_{res}$ [Hz] |
|---|---|---|---|
| 5 | 80 | 80 | 7500 |
| 4 | 51 | 51 | 9375 |
| 3 | 29 | 29 | 12500 |
| 2 | 13 | 13 | 18750 |
| 1 | 3 | 3 | 37500 |

**Fig. 3**

# Fig. 4

# Fig. 5

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/191914 A1 (STESSMAN NICHOLAS J [US]) 16 August 2007 (2007-08-16) | 1,4-8, 11,12 | INV. A61N1/37 |
| Y | * paragraphs [0015] - [0018], [0024] - [0025], [0028] - [0029], [0032]; figures 1-8 * | 2,3,9,10 | ADD. A61N1/08 A61N1/36 |
| Y | US 2018/071522 A1 (FELDMAN EMANUEL [US] ET AL) 15 March 2018 (2018-03-15) * paragraphs [0021] - [0022], [0027] - [0031], [0037] - [0048]; figures 3-9 * | 2,3,9,10 | G01R33/28 A61B5/055 |
| Y | US 2008/154342 A1 (DIGBY DENNIS [US] ET AL) 26 June 2008 (2008-06-26) * paragraphs [0008] - [0021], [0066] - [0075]; claims 1-3; figures 10, 11 * | 2,3,9,10 | |
| Y | US 2012/194191 A1 (JENISON TROY A [US]) 2 August 2012 (2012-08-02) * paragraphs [0059] - [0069]; figures 1-6 * | 9,10 | |
| Y | US 2014/039568 A1 (LEGAY THIERRY [FR]) 6 February 2014 (2014-02-06) * paragraphs [0025] - [0036], [0049] - [0050]; figures 1-2 * | 9,10 | TECHNICAL FIELDS SEARCHED (IPC)<br><br>A61N G01R A61B |
| Y | US 2006/293591 A1 (WAHLSTRAND JOHN D [US] ET AL) 28 December 2006 (2006-12-28) * paragraphs [0012] - [0013], [0029] - [0031]; figures 1-4 * | 2,3 | |

The present search report has been drawn up for all claims

| Place of search | Date of completion of the search | Examiner |
|---|---|---|
| Munich | 20 November 2024 | Fischer, Olivier |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 31 5291

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2024

| Patent document cited in search report | | | Publication date | Patent family member(s) | | | Publication date |
|---|---|---|---|---|---|---|---|
| US | 2007191914 | A1 | 16-08-2007 | EP | 1984076 | A1 | 29-10-2008 |
| | | | | JP | 5079711 | B2 | 21-11-2012 |
| | | | | JP | 2009526608 | A | 23-07-2009 |
| | | | | US | 2007191914 | A1 | 16-08-2007 |
| | | | | US | 2009182389 | A1 | 16-07-2009 |
| | | | | WO | 2007094976 | A1 | 23-08-2007 |
| US | 2018071522 | A1 | 15-03-2018 | EP | 3484579 | A1 | 22-05-2019 |
| | | | | US | 2018071522 | A1 | 15-03-2018 |
| | | | | WO | 2018048926 | A1 | 15-03-2018 |
| US | 2008154342 | A1 | 26-06-2008 | EP | 1935450 | A1 | 25-06-2008 |
| | | | | US | 2008154342 | A1 | 26-06-2008 |
| US | 2012194191 | A1 | 02-08-2012 | CN | 103328039 | A | 25-09-2013 |
| | | | | EP | 2667935 | A1 | 04-12-2013 |
| | | | | JP | 6043299 | B2 | 14-12-2016 |
| | | | | JP | 2014504526 | A | 24-02-2014 |
| | | | | US | 2012194191 | A1 | 02-08-2012 |
| | | | | WO | 2012102744 | A1 | 02-08-2012 |
| US | 2014039568 | A1 | 06-02-2014 | EP | 2206532 | A1 | 14-07-2010 |
| | | | | US | 2010176808 | A1 | 15-07-2010 |
| | | | | US | 2014039568 | A1 | 06-02-2014 |
| US | 2006293591 | A1 | 28-12-2006 | US | 2006293591 | A1 | 28-12-2006 |
| | | | | WO | 2006124481 | A2 | 23-11-2006 |

EPO FORM P0459

For more details about this annex : see Official Journal of the European Patent Office, No. 12/82